(19) Europäisches Patentamt
European Patent Office
Office européen des brevets

(11) Veröffentlichungsnummer: **0 165 450**
A2

# (12) EUROPÄISCHE PATENTANMELDUNG

(21) Anmeldenummer: 85105796.8

(22) Anmeldetag: 11.05.85

(51) Int. Cl.⁴: **A 61 K 31/44**
A 61 K 45/06, A 61 K 31/535
//(A61K31/44, 31:165),
(A61K31/44, 31:135),
(A61K31/44, 31:40),
(A61K31/535, 31:44)

(30) Priorität: 23.05.84 DE 3419130

(43) Veröffentlichungstag der Anmeldung:
27.12.85 Patentblatt 85/52

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Posanski, Ulrich, Dr.
Ahornweg 65b
D-5064 Rösrath(DE)

(72) Erfinder: Lejeune, Philippe Oliver, Dr.
In den Birken 98 f
D-5600 Wuppertal 1(DE)

(72) Erfinder: Porges, Eduard, Dr.
Ahornstrasse 13
D-5000 Köln 71(DE)

(72) Erfinder: Rämsch, Klaus-Dieter, Dr.
Scheidtstrasse 141
D-5600 Wuppertal 21(DE)

(72) Erfinder Hegasy, Ahmed, Dr.
Tempelhofer Strasse 57
D-5090 Leverkusen 1(DE)

## (54) Nifedipinkombinationspräparate und Verfahren zu ihrer Herstellung.

(57) Die Erfindung betrifft neue Nifedipin-Kombinationspräparate in fester Form mit verbesserter Bioverfügbarkeit, enthaltend Nifedipin und β-Blocker, Verfahren zu ihrer Herstellung und ihre Verwendung als Arzneimittel, insbesondere als Coronar- und Blutdruckmittel.

EP 0 165 450 A2

BAYER AKTIENGESELLSCHAFT
Konzernverwaltung RP
Patentabteilung

5090 Leverkusen, Bayerwerk

V (Pha)
KS/bo/c

# Nifedipinkombinationspräparate und Verfahren zu ihrer Herstellung

Die Erfindung betrifft neue Nifedipin-Kombinationspräparate in fester Form mit verbesserter Bioverfügbarkeit, enthaltend Nifedipin und ß-Blocker, Verfahren zu ihrer Herstellung und ihre Verwendung als Arzneimittel, insbesondere als Coronar- und Blutdruckmittel.

Es ist bereits bekannt geworden, daß Nifedipin sehr starke kreislaufbeeinflussende Wirkungen besitzt (vgl. britisches Patent 1 173 862). Aufgrund der schweren Löslichkeit von Nifedipin treten bei der galenischen Zubereitung von Arzneispezialitäten eine Reihe von Schwierigkeiten auf, wie aus zahlreichen Patenten und Patentanmeldungen für spezielle Formulierungen dieses Wirkstoffes ersichtlich wird. So betrifft z.B. das US-Patent 3 784 684 spezielle Nifedipin enthaltende Gelatinebeißkapseln, durch welche die Coronarwirkung von Nifedipin vorteilhaft genutzt werden kann. In dem britischen Patent 1 456 618 werden weiterhin feste Arzneizubereitungen beschrieben und beansprucht, welche ebenfalls eine gute Bioverfügbarkeit von

Nifedipin gewährleisten sollen. Auch in der DT-OS 2 822 882 werden feste Arzneiformen beschrieben, bei denen durch den Einsatz bestimmter Lösungsvermittler und oberflächenaktiver Substanzen die Schwerlöslichkeit des Wirkstoffs kompensiert werden soll. Auch in der europäischen Offenlegungsschrift 1 247 soll die Resorbierbarkeit von Nifedipin durch die Verwendung von Polyethylenglykol und bestimmter poröser Trägersubstanzen verbessert werden.

In der Literatur werden ebenfalls verschiedene Methoden zur Verbesserung der Bioverfügbarkeit, die unmittelbar mit einer verbesserten Löslichkeit verbunden ist, beschrieben. Neben der Änderung der chemischen Struktur, z.B. durch Einbau von löslichkeitsverbessernden Substituentengruppen sind zahlreiche Methoden beschrieben, die eine Änderung der physikalischen Eigenschaften des Wirkstoffes betreffen. So wird z.B. die Zerkleinerung von Wirkstoffpartikeln, Veränderung der Kristallstruktur, die Herstellung von Salzformen, Zugabe von Netzmitteln, Komplexbildung mit anderen Substanzen, Verwendung von biologischen Carriern, Erstellung fester Dispersionen (Copräzipitate) oder die Herstellung von Oberflächenadsorbaten vorgeschlagen (vgl. S.H. Yalkowskay, Drugs and the pharmaceutical science, 12, 135-180 (1981) und J. Polderman, Formulation and preparation of dosage forms, Elsevier/North-Holland Biomedical Press, 1977, 215-219).

Alle bisherigen Versuche, die schlechte Löslichkeit von Nifedipin durch bestimmte Maßnahmen zu kompensieren und gleichzeitig eine gute Bioverfügbarkeit zu gewährleisten, besitzen eine Reihe von Nachteilen. Der Einsatz von oberflächenaktiven Substanzen, Lösungsvermittlern und bestimmten Trägerstoffen, die eine besondere Oberfläche haben, z.B. porös sind, führt häufig zu Verabreichungsformen, bei denen die Präparate unerwünscht groß sind. Zur Erleichterung des Schluckens werden solche Tabletten oder Kapseln häufig in spezifische Formen wie z.B. Ellipsoide oder Längsformen überführt, was jedoch bei Präparaten mit einem Gewicht über 400 mg auch nicht immer zu befriedigenden Ergebnissen führt. Auch das häufigere Einnehmen von kleineren Präparaten stellt keine befriedigende Lösung dar.

Für Arzneizubereitungen gilt, daß sowohl die Zahl als auch die Menge der Hilfs- und Trägerstoffe möglichst niedrig gehalten werden soll. Beim Vergleich zweier Arzneispezialitäten wird man immer demjenigen Präparat den Vorzug geben, welches neben dem Wirkstoff möglichst wenig Hilfsstoffe und Zuschlagstoffe enthält, um unerwünschte biologische Nebenwirkungen weitgehend zu vermeiden.

Die vorliegende Erfindung betrifft neue feste Kombinationspräparate mit verbesserter Bioverfügbarkeit, enthaltend 1 Gewichtsteil Nifedipin und 0,5 bis 10 Gewichtsteile eines ß-Blockers und übliche Hilfs- und Trägerstoffe.

Von besonderem Interesse sind erfindungsgemäße Kombinationspräparate, die einen ß-Blocker aus der Gruppe Acebutolol, Atenolol, Nadolol, Propranolol, Pindolol oder Timolol enthalten.

Insbesondere seien Acebutolol und Atenolol genannt.

Von besonderem Interesse sind erfindungsgemäße Kombinationspräparate enthaltend 1 Gewichtsteil Nifedipin, und 1 bis 5 Gewichtsteile Acebutolol oder Atenolol.

Die Herstellung der erfindungsgemäßen Kombinationspräparate erfolgt, indem man Nifedipin und den ß-Blockerwirkstoff mit bekannten Hilfs- und Trägerstoffen gemeinsam mittels eines Feuchtgranulationsverfahrens oder eines Trockenkompaktierverfahrens granuliert und gegebenenfalls mit mindestens einem Tablettenfüllstoff zu Tabletten verpreßt.

Die Herstellung des erfindungsgemäßen verwendeten Nifedipins erfolgt vorzugsweise durch Umsetzung einer Ylidenverbindung der Formel II

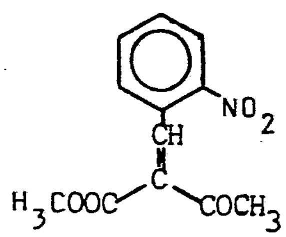

(II)

mit einer Enaminverbindung der allgemeinen Formel III

$$H_3C-\underset{\displaystyle NH_2}{\underset{|}{C}}=CH-COOCH_3 \qquad (III)$$

in niederen Alkoholen mit 1 bis 4 Kohlenstoffatomen bei Temperaturen zwischen 40 und 100°C.

Eine bevorzugte Verfahrensvariante besteht darin, daß Reaktionswasser durch azeotrope Destillation zu entfernen. Die Verbindungen II und III können entweder in molaren Mengen oder mit einem geringen Überschuß der Enaminverbindung III eingesetzt werden.

Die Herstellung der Ylidenverbindung II erfolgt durch Reaktion molarer Mengen eines Ketocarbonsäureesters mit o-Nitrobenzaldehyd in Gegenwart von niederen Alkoholen mit 1 bis 4 C-Atomen als Lösungsmittel und in Gegenwart von katalytischen Mengen Piperidinacetat bei Temperaturen zwischen 20 und 60°C. Die Enaminverbindung der Formel III wird hergestellt durch Umsetzung eines Ketocarbonsäureesters mit Ammoniak in einem niederen Alkohol (1-4 C-Atome), vorzugsweise in Isopropanol, bei Temperaturen zwischen 0 und 40°C. Der Reaktionspartner Ammoniak kann auch in wäßriger Lösung eingesetzt werden.

Als Hilfsstoffe seien beispielhaft genannt:
Zerfallsförderer wie Stärke, modifizierte Stärke, Zellulose, Zellulosederivate, quervernetztes Polyvinylpyrrolidon (PVPP), Natriumalginat und kolloidales Siliciumdioxid; Bindemittel wie Gelatine, Tragant, Glukosesirup, Stärkekleister, Polyvinylpyrrolidone (PVP), Zellulosederivate, Polyethylenglykol MG 1000-5000 (PEG), und Alginate; Schmiermittel wie Magnesiumstearat, Calciumstearat, Stearinsäure, Paraffin, Talkum, pflanzliche oder tierische Fette, Öle und Wachse, Polyethylenglykol MG 1000-5000 und Silikone.

Als Füllstoffe seien beispielhaft genannt:
Calciumsulfat, Calciumcarbonat, di- und tribasische Calciumphosphate, Magnesiumcarbonat, Magnesiumhydroxycarbonat, Natriumchlorid, Natrium- und Kalium-zitrate, Tartrate und Succinate, Stärke, modifizierte Stärke, Zellulose, Zellulosepulver, Zucker wie Lactose, Saccharose oder Dextrose und Zuckeralkohole wie Mannit oder Sorbit.

Von besonderem Interesse sind Hilfs- und Füllstoffe aus der Gruppe Stärke, modifizierte Stärke wie Stärkekleister, Zellulose, PVP, PVPP, kolloidales Siliciumdioxid, Lactose, Magnesiumhydroxycarbonat, Magnesiumstearat und Calciumstearat.

Bei Kenntnis des Standes der Technik ist es ausgesprochen überraschend, daß durch die gemeinsame Granulation von Nifedipin mit einem der genannten ß-Blockern eine signifikante Erhöhung der Freisetzung und damit eine signifikante Verbesserung der Bioverfügbarkeit für Nifedipin erreicht werden kann. Dieser Effekt ist nicht abhängig von der Anwesenheit zusätzlicher Hilfsstoffe, die nach Kenntnis nach Standes der Technik die Freisetzungsrate erhöhen wie z.B. Komplexbildnern, oberflächenaktiven Stoffen oder wasserlöslichen Polymeren.

Im Freisetzungstest nach der USP-Paddle-Methode unter den folgenden Bedingungen

900 ml 0,1 N-Salzsäure
Paddle-Drehzahl 100 U/Min.

wurden nach 1 Stunde Freisetzungsraten für Nifedipin von 85 % erreicht für erfindungsgemäße Kombinationspräparate (Produkt gemäß Beispiel 1). Eine entsprechende Zubereitung einer herkömmlichen Nifedipintablette, in welcher das Nifedipin in gleicher kristalliner Zusammensetzung

eingesetzt wurde und die die gleichen Hilfsstoffe enthält, zeigt nach 1 Stunde nur eine Freisetzungsrate von unter 40 %. Der Verlauf der Freisetzung ist aus der Tabelle 1 ersichtlich.

Tabelle 1

In vitro - Freisetzung von nifedipinhaltigen Tabletten (USP-Paddle-Methode)

| Freisetzungszeit /h/ | kumulative Freisetzung von Nifedipin in Prozent der eingesetzten Dosis<br>Tablette Beispiel 1 | Tablette Vergleichsbeispiel |
|---|---|---|
| 0,25 | 49 | 19 |
| 0,5 | 76 | 28 |
| 1 | 85 | 39 |

Vergleichsbeispiel

100 g Nifedipin, 2080 g Maisstärke, 1150 g Cellulosepulver und 50 g Milchzucker werden in einem Planetenmischer 5 Minuten gemischt und mit einer wäßrigen Polyvinylpyrrolidon/Tween 80-Lösung (Feststoffgehalt 200 g/5 g) 15 Minuten granuliert. Die feuchte Masse wird durch eine Raspel (2,0 mm Ø Siebeinsatz) gegeben und in einem Wirbelschichttrockner auf einen Endwassergehalt von ca. 5 % getrocknet.

Das getrocknete Granulat wird durch Siebung (1,0 mm-Siebmaschenweite) egalisiert und intensiv mit 15 g Magnesiumstearat vermischt. Anschließend wird das Granulat zu 360 mg schweren Tabletten verpreßt.

Ausführungsbeispiele:

Beispiel 1

100 g Nifedipin, 1508 g Acebutolol-HCl, 1372 g Maisstärke, 100 g Milchzucker und 400 g Cellulosepulver werden in einem Planetenmischer 5 Minuten gemischt und mit einer wäßrigen Polvinylpyrrolidon/Tween 80-Lösung (Feststoffgehalt 200 g/5 g) 15 Minuten granuliert. Die feuchte Masse wird durch eine Raspel (2,0 mm Ø-Siebeinsatz) gegeben und in einem Wirbelschichttrockner auf einen Endwassergehalt von ca. 4,5 % getrocknet. Das getrocknete Granulat wird durch Siebung (1,0 mm-Siebmaschenweite) egalisiert und intensiv mit 15 g Magnesiumstearat vermischt. Anschließend wird das Granulat zu 360 mg schweren Tabletten verpreßt. Die Tabletten können mit einem Schutzlack überzogen werden.

Beispiel 2

200 g Nifedipin, 2216 g Acebutolol-HCl, 2494 g Maisstärke, 50 g Milchzucker und 900 g Cellulosepulver werden in einem Wirbelschichtgranulator (Glatt WSG 5) erwärmt und zunächst mit einer wäßrigen Natriumlaurylsulfatlösung (Gehalt 10 g) besprüht. Danach wird das Pulver mit einer wäßrigen Hydroxypropylmethylcellulose-Lösung (Gehalt 400 g) granuliert und auf eine Endfeuchte von ca. 4,5 % getrocknet, abgesiebt (Siebweite 0,8 mm Ø) und in einem Kubusmischer mit 20 g Magnesiumstearat vermischt. Das preßfertige Granulat kann zu 314,5 mg schweren Tabletten verarbeitet und in einer Accela-Coater mit einer lichtundurchlässigen Hülle überzogen.

Beispiel 3

1000 g Atenolol, 400 g Nifedipin, 600 g Cellulosepulver, 1200 g Maisstärke, 1200 g schweres Magnesiumcarbonat und 40 g Natriumlaurylsulfat werden in einem Mischgranulator (z.B. Lödige MGT 30) 1 Minute gemischt und 5 Minuten lang mit Stärkekleister (Stärkegehalt 200 g) granuliert bei einer Rotordrehzahl des Granulators von 200 U/Min. Durch Zerhacker-Stufe II wird eine mittlere Teilchengröße von ca. 200 - 300 µ erhalten. Das Granulat wird über eine Raspel (Siebeinsatz 2,0 mm Ø) gegeben und in einem Fließbetttrockner (z.B. Glatt WST 5) bis auf eine Endfeuchte von ca. 4,0 % Wasser getrocknet. Das getrocknete Granulat wird mit 100 g Magnesiumstearat in einem Mischer 10 Minuten gemischt und anschließend zu Tabletten mit einem mittleren Durchmesser von 9 mm und einem Gewicht von ca. 237 mg verpeßt.

Die erhaltenen Tabletten können zusätzlich mit einer lichtundurchlässigen Lackhülle überzogen werden, die z.B. rotes Eisenoxid als Lichtschutz enthält.

Beispiel 4

500 g Atenolol, 200 g Nifedipin, 450 g Cellulosepulver, 400 g Maisstärke, 650 g Calciumcarbonat, 25 g Tween 80 und 50 g Magnesiumstearat werden in einem Kubusmischer gemischt und anschließend trocken kompaktiert (Alexanderwalze WP50; Kompaktierungsdruck 35 bar). Die Schülpen werden vorgebrochen, mit einem 1,25 mm-Sieb egalisiert und mit einer Mischung aus 25 g Magnesiumstearat und 200 g Maisstärke homogen vermischt. Das fertige Granulat wird zu 250 mg schweren Tabletten mit 9 mm Ø verpreßt.

Die erhaltenen Tabletten können direkt verwendet werden oder alternativ noch einen Schutzlack, bestehend aus Hydroxypropylmethylcellulose, Polyethylenglykol und Eisenoxiden oder anderen physiologisch verträglichen Farbpigmenten oder Farblacken erhalten.

Beispiel 5

750 g Atenolol, 300 g Nifedipin, 855 g Maisstärke, 975 g Magnesiumhydroxycarbonat, 150 g vorverkleisterte Maisstärke und 600 g Cellulosepulver werden 8 Minuten in einem Planetenmischer (Collette MP 20) gemischt und 15 Minuten lang, nach kontinuierlicher Zugabe von 250 ml einer 2,0 %igen, wäßrigen Natriumlaurylsulfatlösung, bei erhöhter Drehzahl des Mischers granuliert. Die feuchte Masse wird geraspelt, in einem Wirbelschichttrockner auf ca. 3,8 % Endfeuchte getrocknet und durch Siebung (1,0 mm Maschenweite) egalisiert. Die Endmischung wird nach Untermischung von 75 g Magnesiumstearat erhalten und zu 247 mg schweren Tabletten verpreßt.

Die erhaltenen Tabletten können zusätzlich mit einer tageslichtundurchlässigen Hülle überzogen werden. Das Granulat kann alternativ in Hartgelatinekapseln abgefüllt werden.

Patentansprüche

1. Festes Kombinationspräparat mit verbesserter Bioverfügbarkeit, enthaltend 1 Gewichtsteil Nifedipin und 0,5 bis 10 Gewichtsteile eines ß-Blockers und gegebenenfalls übliche Hilfs- und Trägerstoffe.

2. Kombinationspräparat gemäß Anspruch 1, enthaltend einen ß-Blocker aus der Gruppe Acebutolol, Atenolol, Nadolol, Propranolol, Pindolol und Timolol.

3. Kombinationspräparat gemäß den Ansprüchen 1 bis 2, enthaltend Acebutolol als ß-Blocker.

4. Kombinationspräparat gemäß den Ansprüchen 1 bis 2, enthaltend Atenolol als ß-Blocker.

5. Kombinationspräparat gemäß Ansprüchen 1 bis 4, enthaltend 1 Gewichtsteil Nifedipin und 1 bis 5 Gewichtsteile des ß-Blockers.

6. Kombinationspräparat gemäß den Ansprüchen 1 bis 5 in Form von Granulat, Tabletten, Lacktabletten oder Dragees.

7. Verfahren zur Herstellung von Kombinationspräparaten gemäß Anspruch 1, dadurch gekennzeichnet, daß man 1 Gewichtsteil Nifedipin und 0,5 bis 10 Gewichtsteile des ß-Blockers gemeinsam mit Hilfs und Trägerstoffen granuliert und gegebenenfalls dieses Granulat zu üblichen Applikationsformen weiterverarbeitet.

8. Verfahren gemäß Anspruch 6, dadurch gekennzeichnet, daß man 1 Gewichtsteil Nifedipin und 1 bis 5 Gewichtsteile ß-Blocker mit Stärke, Cellulose und gegebenenfalls sonstigen üblichen Hilfsstoffen in einem Granulator wäßrig granuliert und das erhaltene Granulat ebenfalls in eine übliche Applikationsform überführt.

9. Verfahren zur Herstellung von Kombinationspräparaten gemäß Anspruch 1, dadurch gekennzeichnet, daß man die Ylidenverbindung der Formel II

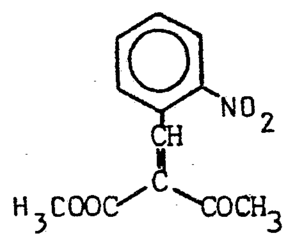

(II)

mit der Enaminverbindung der Formel III

$$H_3C-C(NH_2)=CH-COOCH_3 \qquad (III)$$

in Gegenwart von niederen Alkoholen (1-4 C-Atome), vorzugsweise Isopropanol, als Lösungsmittel bei Temperaturen zwischen 0 und 40°C umsetzt und das so erhaltene Nifedipin gemäß dem Anspruch 7 in eine geeignete Applikationsform überführt.

10. Verwendung von Kombinationspräparaten gemäß Anspruch 1 bei der Bekämpfung von Kreislauferkrankungen.